# EUROPÄISCHE PATENTSCHRIFT

(11) **EP 0 589 301 B1**
(45) Veröffentlichungstag und Bekanntmachung des Hinweises auf die Patenterteilung: **26.06.2002**
(21) Anmeldenummer: 93114540.3
(22) Anmeldetag: 10.09.1993
(51) Int. Cl.: C07D 277/56, C07D 231/14, C07D 333/38, C07D 335/02, A01N 43/78, A01N 43/10, A01N 43/18, A01N 43/56

(54) **Carbonsäureanilide, Verfahren zu ihrer Herstellung und sie enthaltende Mittel zur Bekämpfung von Schadpilzen**
Carboxynilides derivatives, process for their preparation and fungicidal compositions containing them
Carboxanilides, procédé de préparation et compositions fungicides les contenant

(30) Priorität: 21.09.1992 DE 4231517
(43) Veröffentlichungstag der Anmeldung: 30.03.1994
(73) Patentinhaber: BASF Aktiengesellschaft, 67063 Ludwigshafen (DE)
(72) Erfinder: Eicken, Karl, Dr., D-6706 Wachenheim (DE); Koenig, Hartmann, Dr., D-6703 Limburgerhof (DE); Ammermann, Eberhard, Dr., d-6148 Heppenheim (DE); Lorenz, Gisela, Dr., D-6730 Neustadt (DE)

(56) Entgegenhaltungen:
- EP-A- 0 371 950
- EP-A- 0 545 099
- WO-A-91/01311
- WO-A-93/11117
- FR-A- 1 546 183
- FR-A- 2 337 997
- CHEMICAL ABSTRACTS, vol. 81, no. 19, 11. November 1974, Columbus, Ohio, US; abstract no. 115750j, ABDEL-LATEEF ET AL 'Systemic and chemotherapeutic fungicidal activity-chemical structure relation of some 4-methyl-5-thiazolecarboxylic acid derivatives.Laboratory screening tests' Seite 142 ; & ACTA PHYTOPATHOL. Bd. 8, Nr. 3 , 1973 Seiten 269 - 282
- CHEMICAL ABSTRACTS, vol. 94, no. 15, 13. April 1981, Columbus, Ohio, US; abstract no. 115825f, G. A. WHITE ET AL 'Thiophene carboxamide fungicides' Seite 187 ; & PESTIC. BIOCHEM. PHYSIOL Bd. 14, Nr. 1 , 1980 Seiten 26 - 40
- PESTICIDE BIOCHEMISTRY AND PHYSIOLOGY Bd. 25, Nr. 2 , April 1986 , NEW YORK Seiten 188 - 204 G.A. WHITE ET AL 'Thiophene carboxamide fungicides'

## Beschreibung

Die vorliegende Erfindung betrifft Carbonsäureanilide der Formel I 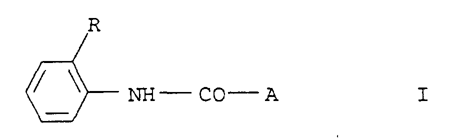 in der die Substituenten die folgende Bedeutung haben:
- R: C₃-C₁₂-Alkyl, C₂-C₁₂-Alkoxy, C₃-C₁₂-Alkenyl, C₃-C₁₂-Alkenyloxy, C₃-C₆-Alkinyl, C₃-C₆-Alkinyloxy, wobei diese Gruppen partiell oder vollständig halogeniert sein können;
Phenyl, welches ein bis fünf Halogenatome und/oder ein bis drei der folgenden Reste tragen kann: C₁-C₄-Alkyl, C₁-C₄-Hal genalkyl, C₁-C₄-Alkoxy, C₁-C₄-Halogenalkoxy, C₁-C₄-Alkylthio oder C₁-C₄-Halogenalkylthio;
- A: ein cyclischer Rest der Formeln A5: 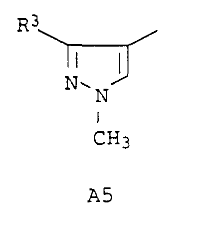 wobei
- R³: C₁-C₄-Alkyl oder C₁-C₄-Halogenalkyl bedeutet.

Außerdem betrifft die Erfindung Verfahren zur Herstellung dieser Verbindungen, sie enthaltende Mittel und Verfahren zu deren Verwendung zur Bekämpfung von Schadpilzen, insbesondere Botrytis.

Aus der Literatur sind N-(2-Methylphenyl)-3-methylthiophen-2-carbonsäureamid, N-(2-Methylphenyl)-2,5-dimethylthiophen-3-carbonsäureamid und N-(2-Methylphenyl)-1,3,5-trimethylpyrrazol-4-carbonsäureamid als fungizide Wirkstoffe bekannt (DE-A 27 01 091; Pestic. Biochem. Physiol., 25(2), 188-204 (1986)).

Außerdem werden in der nachveröffentlichten Anmeldung WO-A 93/11,117 die vom den Verbindungen I ausgenommenen Pyrazolcarbonsäureanilide als fungizid wirksam beschrieben.

Aufgabe der vorliegenden Erfindung waren neue fungizid wirksame Verbindungen mit verbessertem Wirkungsspektrum.

Demgemäß wurden die eingangs definierten Verbindungen I gefunden.

Außerdem wurden Verfahren zur Herstellung dieser Verbindungen, sie enthaltende Mittel und Verfahren zu deren Verwendung zur Bekämpfung von Schadpilzen gefunden.

Ma erhält die Verbindungen I im allgemeinen dadurch, daß man ein Carbonsäurehalogenid der Formel II in an sich bekannter Weise (z.B. J. March, Advanced Organic Chemistry, 2nd Ed., 382 f, McGraw-Hill, 1977) in Gegenwart einer Base mit einem Anilin der Formel III umsetzt.

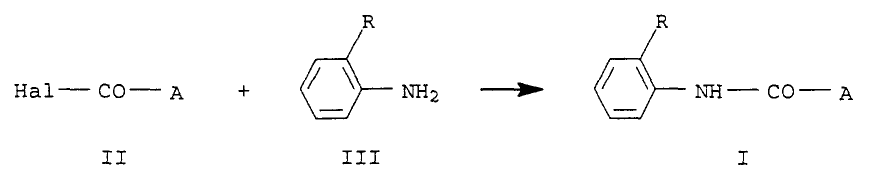

Der Rest Hal in der Formel II steht für ein Halogenatom wie Chlor, Brom und Jod, insbesondere Chlor oder Brom. Diese Umsetzung erfolgt üblicherweise bei Temperaturen von -20 °C bis 100 °C, vorzugsweise 0 °C bis 50 °C.

Geeignete Lösungsmittel sind aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, und metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Triethylamin und Pyridin verwendet.

Die Basen werden im allgemeinen in äquimolarem Mengen bezogen auf die Verbindung II eingesetzt. Sie können aber auch in einem Überschuß von 5 mol-% bis 30 mol-%, vorzugsweise 5 mol-% bis 10 mol-%, oder - im Falle der Verwendung von tertiären Aminen - gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß von 1 mol-% bis 20 mol-%, vorzugsweise 1 mol-% bis 10 mol-%, bezogen auf III einzusetzen.

Die für die Herstellung der Verbindungen I benötigten Ausgangsstoffe der Formel II und III sind bekannt oder können analog zu den bekannten Verbindungen synthetisiert werden (Helv. Chim. Acta, 60, 978 (1977); Zh. Org. Khim., 26, 1527 (1990); Heterocycles 26, 1885 (1987); Izv. Akad. Nauk. SSSR Ser. Khim., 2160 (1982); THL 28, 593 (1987); THL 29, 5463 (1988)).

Im Hinblick auf ihre Verwendung in fungiziden Mitteln kommen Verbindungen der Formel I in Betracht, in der die Substituenten die folgende Bedeutung haben:
- R: C₂-C₁₂-Alkyl wie Ethyl und geradkettiges oder verzweigtes Propyl, Butyl, Pentyl, Hexyl, Heptyl, Octyl, Nonyl, Decyl, Undecyl und Dodecyl, besonders geradkettiges oder verzweigtes C₃-C₁₀-Alkyl wie Propyl, 1-Methylethyl, Butyl, 1-Methylpropyl, 2-Methylpropyl, 1,1-Dimethylethyl, n-Pentyl, 1-Methylbutyl, 2-Methylbutyl, 3-methylbutyl, 1,2-Dimethylpropyl, 1,1-Dimethylpropyl, 2,2-Dimethylpropyl, 1-Ethylpropyl, n-Hexyl, 1-Methpentyl, 2-Methylpentyl, 3-Methylpentyl, 4-Methylpentyl, 1,2-Dimethylbutyl, 1,3-Dimethylbutuyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl, insbesondere Propyl, 1-Methylethyl, Butyl, 1-Methylbutyl, 2-Methylbutyl, 1,1-Dimethylethyl, Pentyl, 1-Methylbutyl, Hexyl, Heptyl und 1-Methylheptyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
C₂-C₁₂-Alkoxy wie Ethoxy und geradkettiges oder verzweigtes Propyloxy, Butyloxy, Pentyloxy, Hexyloxy, Heptyloxy, Octyloxy, Nonyloxy, Decyloxy, Undecyloxy und Dodecyloxy, besonders geradkettiges oder verzweigtes C₂-C₁₀-Alkoxy wie Ethoxy, Propoxy, 1-Methylethoxy, Butoxy, 1-Methylpropoxy, 2-Methylpropoxy, 1,1-Dimethylethoxy, n-Pentyloxy, 1-Methylbutoxy, 2-Methylbutoxy, 3-Methylbutoxy, 1,2-Dimethylpropoxy, 1-Ethylpropoxy, n-Hexyloxy, 1-Methylpentyloxy, 2-Methylpentyoxy, 3-Methylpentyloxy, 4-Methylpentyloxy, 1,2-Dimethylbutoxy, 1,3-Dimethylbutoxy, 2,3-Dimethylbutoxy, 1,2-Dimethylbutoxy, 2,2-Dimethylbutoxy, 3,3-Dimethylbutoxy, 1,1,2-Trimethylpropoxy, 1,2,2-Trimethylpropoxy, 1-Ethylbutoxy, 2-Ethylbutoxy, 1-Ethyl-2-methylpropoxy, n-Heptyloxy, 1-Methylhexyloxy, 2-Methylhexyloxy, 3-Methylhexyloxy, 4-Methylhexyloxy, 5-Methylhexyloxy, 1-Ethylpentyloxy, 2-Ethylpentyloxy, 1-Propylbutoxy, Octyloxy, 1-Methylheptyloxy, 2-Methylheptyloxy, 1-Ethylhexyloxy, 2-Ethylhexyloxy, 1-Propylpentyloxy, 2-Propylpentyloxy, Nonyloxy, 1-Methyloctyloxy, 2-Methyloctyloxy, 1-Ethylheptyloxy, 2-Ethylheptyloxy, 1-Propylhexyloxy, 2-Propylhexyloxy, Decyloxy, 1-Methylnonyloxy, 2-Methylnonyloxy, 1-Ethyloctyloxy, 2-Ethyloctyloxy, 1-Propylheptyloxy und 2-Propylheptyloxy, insbesondere Ethoxy, Propyloxy, 1-Methylethoxy, Butyloxy, 1-Methylpropyloxy, 2-Methylpropyloxy, 1,1-Dimethylethoxy, Pentyloxy, Hexyloxy und 2-Ethylhexyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;
C₃-C₁₂-Alkenyl wie geradkettiges oder verzweigtes Propenyl, Butenyl, Pentenyl, Hexenyl, Heptenyl, Octenyl, Nonenyl, Decenyl, Undecenyl und Dodecenyl, besonders gradkettiges oder verzweigtes C₃-C₁₀-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-2-propenyl, 1-Methyl-2-pentenyl, 2-Methyl-2-pentenyl, 1-Methyl-3-pentenyl, 2-Methyl-3-pentenyl, 1-Methyl-2-hexenyl, 2-Methyl-2-hexenyl, 1-Methyl-3-hexenyl, 2-Methyl-3-hexenyl, 1-Ethyl-2-pentenyl, 2-Ethyl-2-pentenyl, 1-Ethyl-3-pentenyl, 2-Ethyl-3-pentenyl, 1-Methyl-2-heptenyl, 2-Methyl-2-heptenyl, 1-Methyl-3-heptenyl, 2-Methyl-3-heptenyl, 1-Ethyl-2-hexenyl, 2-Ethyl-2-hexenyl, 1-Ethyl-3-hexenyl, 2-Ethyl-3-hexenyl, 1-Methyl-2-octenyl, 2-Methyl-2-octenyl, 1-Methyl-3-octenyl, 2-Methyl-3-octenyl, 1-Ethyl-2-heptenyl, 2-Ethyl-2-heptenyl, 1-Ethyl-3-heptenyl, 2-Ethyl-3-heptenyl, 1-Ethyl-2-octenyl, 2-Ethyl-2-octenyl, 1-Ethyl-3-octenyl und 2-Ethyl-3-octenyl, insbesondere 1-Propenyl, 2-Propenyl, 1-Methylethenyl, 1-Methyl-2-propenyl, 2-Methyl-2-propenyl, 1-Ethyl-2-propenyl, 1-Methyl-2-butenyl, 1-Ethyl-2-butenyl, 1-(1-Methylethyl)-2-butenyl, 1-Butyl-2-butenyl, 1-Methyl-2-pentenyl und 1,4-Dimethyl-2-pentenyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, insbesondere 3-Chlor-2-propenyl, 2,3-Dichlor-2-propenyl, 2,3,3-Trichlor-2-propenyl;
C₃-C₁₂-Alkenyloxy wie geradkettiges oder verzweigtes Propenyloxy, Butenyloxy, Pentenyloxy, Hexenyloxy, Heptenyloxy, Octenyloxy, Nonenyloxy, Decenyloxy, Undecenyloxy und Dodecenyloxy, besonders gradkettiges oder verzweigtes C₃-C₁₀-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-2-propenyloxy, 1-Methyl-2-pentenyloxy, 2-Methyl-2-pentenyloxy, 1-Methyl-3-pentenyloxy, 2-Methyl-3-pentenyloxy, 1-Methyl-2-hexenyloxy, 2-Methyl-2-hexenyloxy, 1-Methyl-3-hexenyloxy, 2-Methyl-3-hexenyloxy, 1-Ethyl-2-pentenyloxy, 2-Ethyl-2-pentenyloxy, 1-Ethyl-3-pentenyloxy, 2-Ethyl-3-pentenyloxy, 1-Methyl-2-heptenyloxy, 2-Methyl-2-heptenyloxy, 1-Methyl-3-heptenyloxy, 2-Methyl-3-heptenyloxy, 1-Ethyl-2-hexenyloxy, 2-Ethyl-2-hexenyloxy, 1-Ethyl-3-hexenyloxy, 2-Ethyl-3-hexenyloxy, 1-Methyl-2-octenyloxy, 2-Methyl-2-octenyloxy, 1-Methyl-3-octenyloxy, 2-Methyl-3-octenyloxy, 1-Ethyl-2-heptenyloxy, 2-Ethyl-2-heptenyloxy, 1-Ethyl-3-heptenyloxy, 2-Ethyl-3-heptenyloxy, 1-Ethyl-2-octenyloxy, 2-Ethyl-2-octenyloxy, 1-Ethyl-3-octenyloxy und 2-Ethyl-3-octenyloxy, insbesondere 2-Propenyloxy, 1-Methyl-2-propenyloxy, 2-Methyl-2-propenyloxy, 2-Pentenyloxy, 3-Pentenyloxy, 1-Methyl-2-butenyloxy und 1-Methyl-2-pentenyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, insbesondere 3-Chlor-2-propenyloxy, 2,3-Dichlor-2-propenyloxy und 2,3,3-Trichlor-2-propenyloxy;
C₃-C₆-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,2-Dimethyl-2-butinyl, 1,1-Dimethyl-3-butinyl, 1,2-Dimethyl-3-butinyl, 2;2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl, 2-Butinyl und 3-Butinyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise 3-Chlor-2-propinyl, 3-Chlor-2-butinyl und 4-Chlor-3-butinyl;
C₃-C₆-Alkinyloxy wie 2-Propinyloxy, 2-Butinyloxy, 3-Butinyloxy, 1-Methyl-2-propinyloxy, 2-Pentinyloxy, 3-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propionyloxy, 1-Ethyl-2-propinyloxy, 2-Hexinyloxy, 3-Hexinyloxy, 4-Alkinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-3-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise 2-Propinyloxy, 2-Butinyloxy, 1-Methyl-2-propinyloxy und 1-Methyl-2-butinyloxy, 2-Propinyloxy, 2-Butinyloxy, 3-Butinyloxy und 1-Methyl-2-propinyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise 3-Chlor-2-propinyloxy, 3-Chlor-2-butinyloxy und 4-Chlor-3-butinyloxy;
Phenyl, welches ein bis fünf Halogenatome wie Fluor, Chlor, Brom und Jod, insbesondere Fluor, Chlor und Brom, und/oder ein bis drei der folgenden Reste tragen kann:
- C₁-C₄-Alkyl wie vorstehend genannt;
- C₁-C₄-Halogenalkyl wie vorstehend genannt;
- C₁-C₄-Alkoxy wie vorstehend genannt;
- C₁-C₄-Halogenalkoxy wie vorstehend genannt;
- C₁-C₄-Alkylthio wie Methylthio, Ethylthio, Propylthio, 1-Methylethylthio, Butylthio, 1-Methylpropylthio, 2-Methylpropylthio und 1,1-Dimethylethylthio;
- oder C₁-C₄-Halogenalkylthio, besonders C₁-C₂-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio.
- A: steht für einen cyclischen Rest 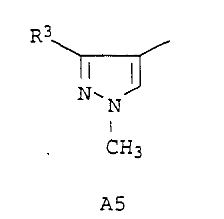 in dem R³ die folgende Bedeutung hat:
- R³: C₁-C₄-Alkyl wie vorstehend genannt oder C₁-C₄-Halogenalkyl wie vorstehend genannt.

Im Hinblick auf die biologische Wirkung besonders bevorzugte Verbindungen der Formel I sind solche, in denen R die vorstehend gegebene Bedeutung hat und A für einen cyclischen Rest A5 steht, wobei
- R³: Methyl
oder C₁-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl bedeutet.

Insbesondere sind solche Verbindungen der Formel I bevorzugt, in denen R die vorstehend gegebene Bedeutung hat und A für einen cyclischen Rest A5 steht,
wobei
- R³: Methyl, Difluormethyl oder Trifluormethyl bedeutet.

Besonders bevorzugte Verbindungen der Formel I, in denen
- R: für iso-Butyl, sek.-Butyl, Cyclopentyl, Cyclohexyl oder Phenyl steht.

Besonders bevorzugt sind ferner Verbindungen I, in denen
- A: für A5 und
- R³: für Methyl oder Trifluormethyl steht,
vorzugsweise Verbindungen I, in denen
- A: für A5,
- R³: für Methyl oder Trifluormethyl und
- R: für iso-Butyl, sek.-Butyl, 2-Ethylbutyl, Cyclopent-2-en-1-yl, Phenyl oder 1,1,2,2-Tetrafluorethoxy steht und
insbesondere Verbindungen I, in denen
- A: für A5,
- R³: für Methyl oder Trifluormethyl und
- R: für iso-Butyl, sek.-Butyl, 2-Ethylbutyl oder 1,1,2,2-Tetrafluorethoxy steht.

Insbesondere bevorzugte Verbindungen der Formel I sind in der folgenden Tabelle A zusammengestellt.

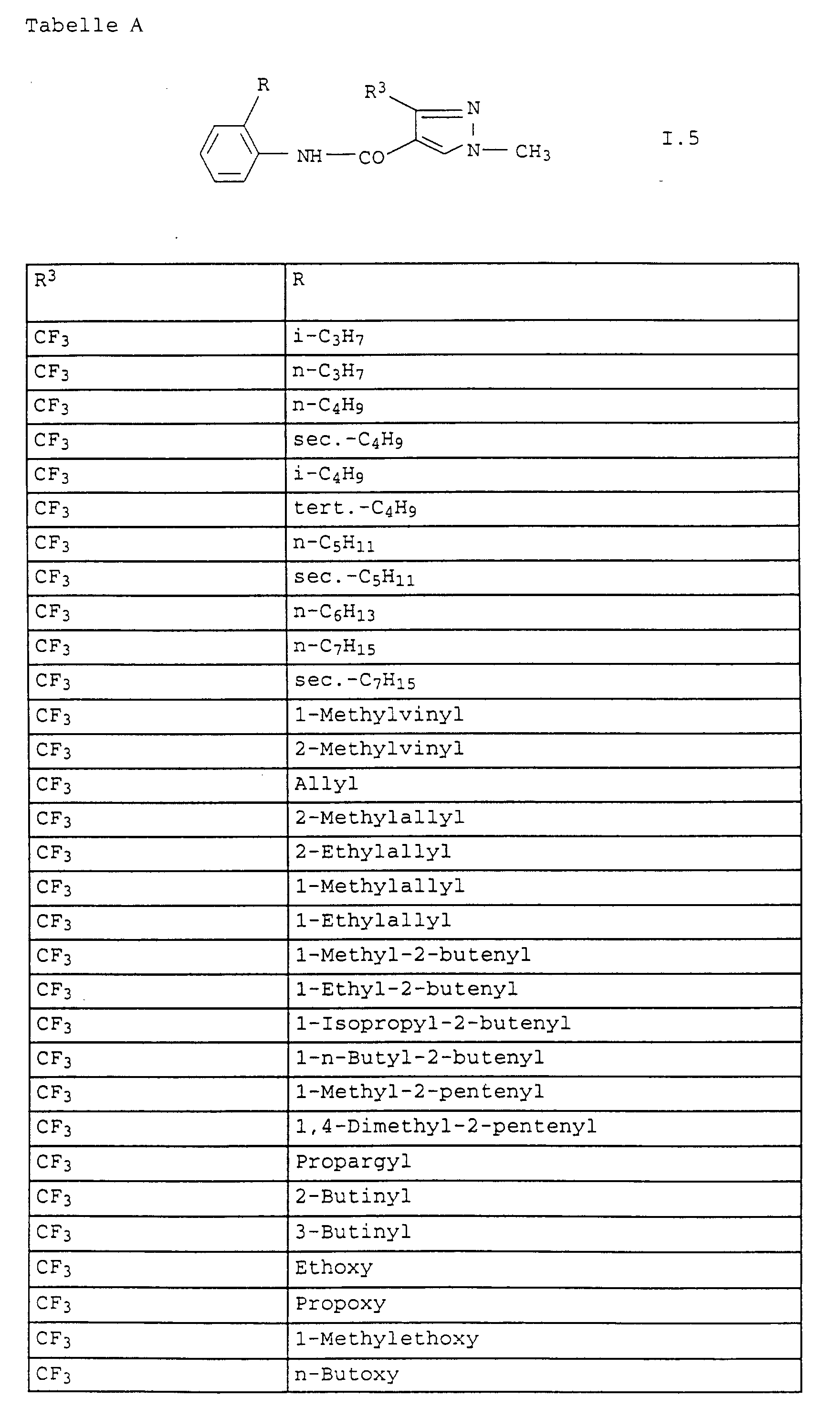

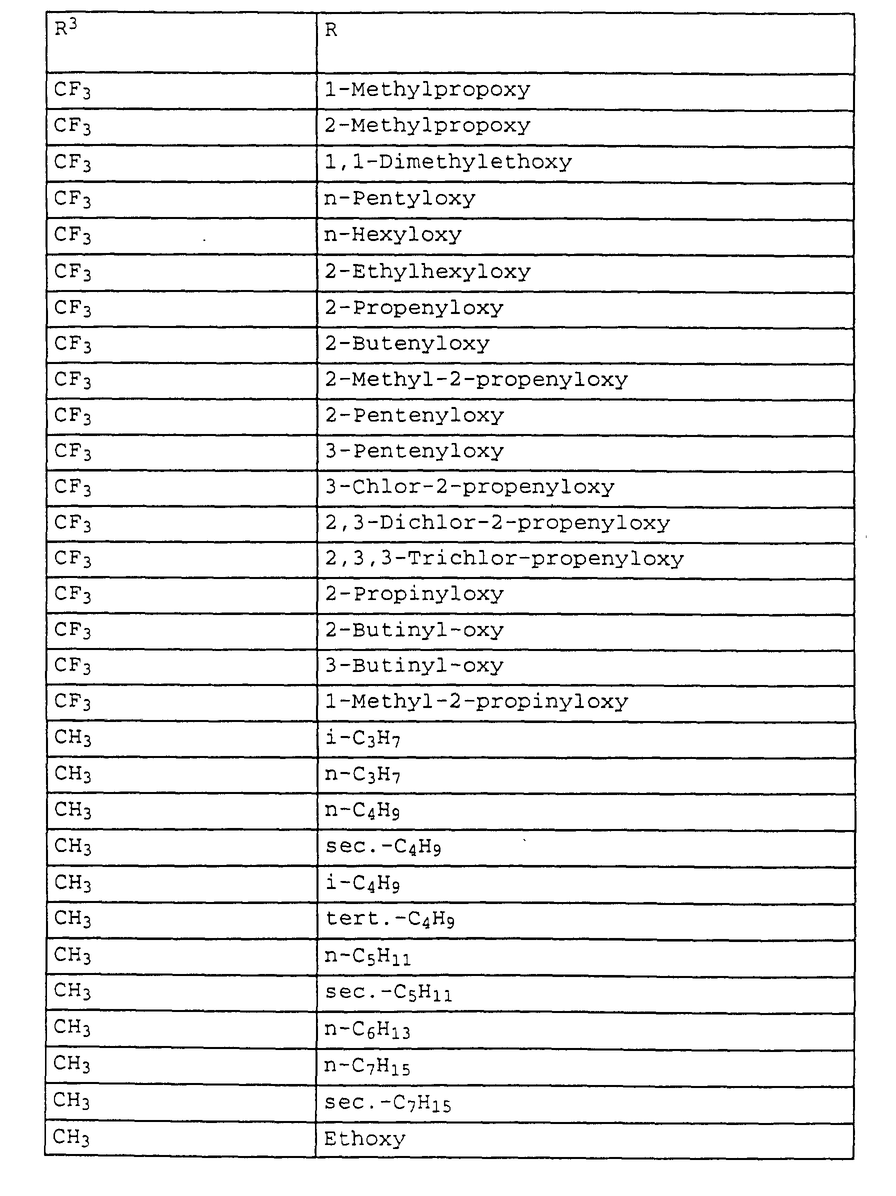

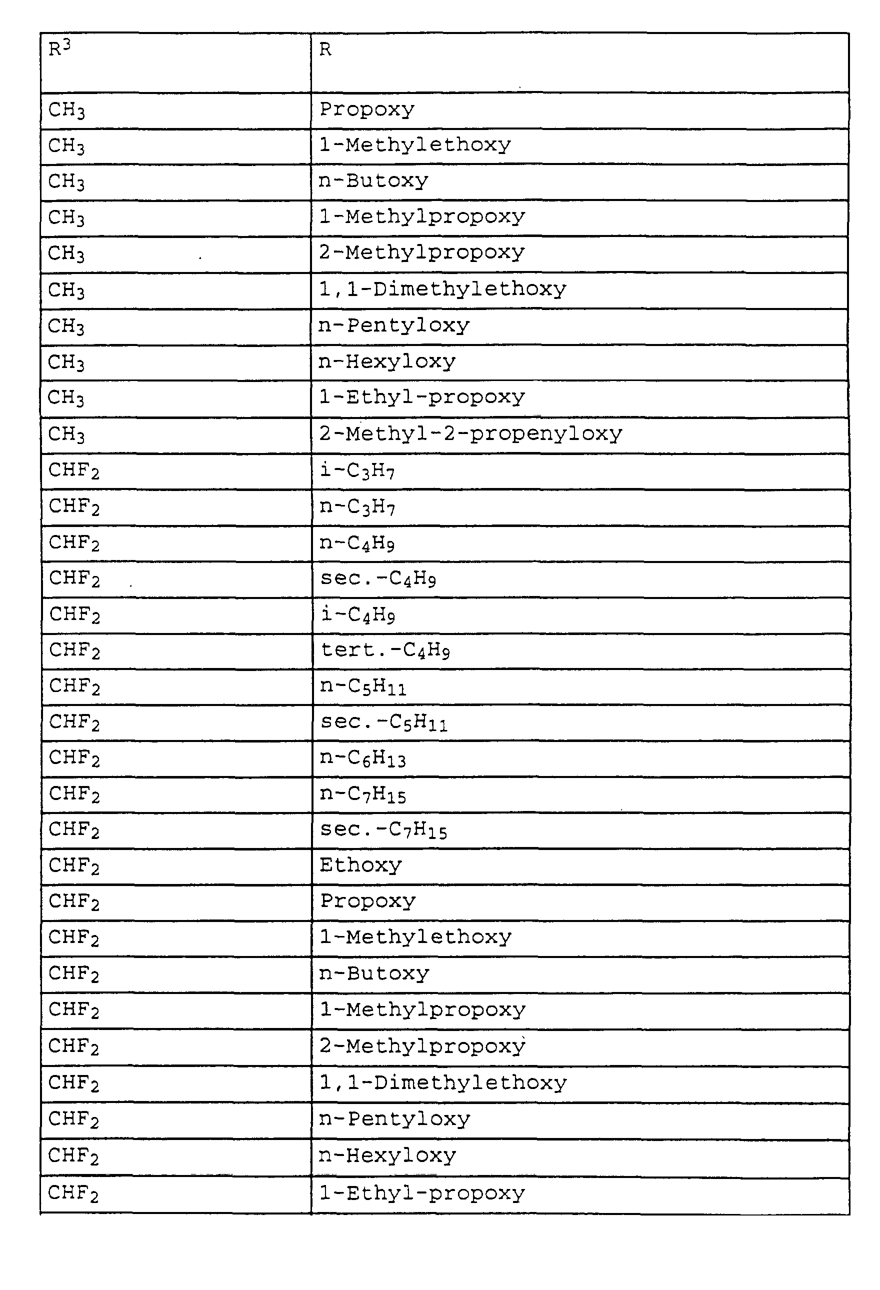

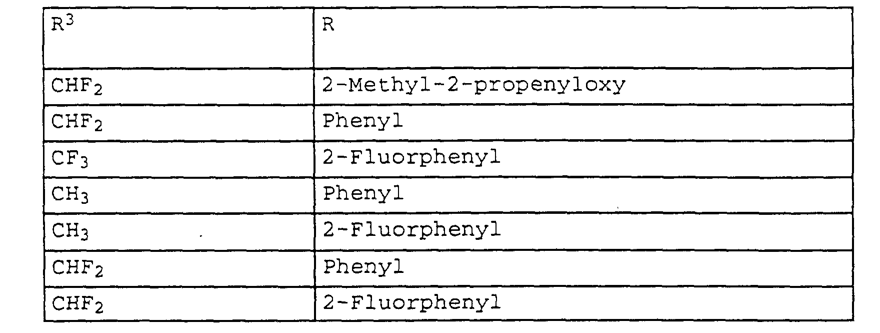

Die neuen Wirkstoffe eignen sich besonders zum Schutz von verschiedenen Materialien gegen den Abbau bzw. die Zerstörung durch Bakterien oder Pilze oder gegen den Befall und Bewuchs durch Mikroorganismen. Materialien, die mit den neuen Wirkstoffen konserviert bzw. mikrozid ausgerüstet werden können, sind beispielsweise Leime und Klebstoffe, Stärkelösungen, Wachsemulsionen, Tonemulsionen, Schlichten, Appreturen, Spinnbäder, Gelatinezubereitungen, Fensterkitt, Fugendichtungsmassen, Kühlschmierstoffe, Bohröle, Treibstoffe, Kunststoffdispersionen, Dispersionsfarben, Textilien, Leder, Rohhäute und Kosmetika. Weiterhin sind die Verbindungen als Schleimbekämpfungsmittel in der Papierindustrie, in Rückkühlwerken und in Luftbefeuchtungsanlagen geeignet.

Des weiteren eignen sich die Verbindungen I zum Schutz folgender Pflanzenarten vor dem Befall durch Mikroorganismen:

Getreide (z.B. Weizen, Gerste, Roggen, Hafer, Reis, Sorhum und Verwandte); Rüben (z.B. Zucker- und Futterrüben); Kern-, Steinund Beerenobst (z.B. Äpfel, Birnen, Pflaumen, Pfirsiche, Mandeln, Kirschen, Erdbeeren, Himbeeren und Brombeeren); Hülsenfrüchte (z.B. Bohnen, Linsen, Erbsen, Soja); Ölkulturen (z.B. Raps, Senf, Mohn, Oliven, Sonnenblumen, Kokos, Rizinus, Kakao, Erdnüsse); Gurkengewächse (z.B. Kürbis, Gurken, Melonen); Fasergewächse (z.B. Baumwolle, Flachs, Hanf, Jute); Citrusfrüchte (z.B. Orangen, Zitronen, Pampelmusen, Mandarinen); Gemüsesorten (z.B. Spinat, Kopfsalat, Spargel, Kohlarten, Möhren, Zwiebeln, Tomaten, Kartoffeln, Paprika); Lorbeergewächse (z.B. Avocado, Cinnamonum, Kampfer) oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover corps).

Folgende Mikroorganismen lassen sich beispielsweise mit den neuen Verbindungen I bekämpfen:

Straphylococcus aureus, Escherichia coli, Klebsielle pneumoniae, Citrobacter freundii, Proteus vulgaris, Pseudomonas aeruginosa, Desulfovibrio desulfuricans, Streptoverticillium rubrireticuli, Aspergillus niger, Aspergillus versicolor, Penicillium funiculosum, Penicillium expansum, Penicillium glaucum, Paecilomyces variotii, Trichoderma viride, Chaetomium globosum, Aspergillus amstelodami, Phoma pigmentovora, Phoma violacea, Aureobasidium pullulans, Saccharomyces cerevisiae, Alternaria tenuis, Stemphylium macrosporoideum, Cladosporium herbarum, Cladosporium resinae, Candida albicans, Trichophyton mentagrophytes, Geotrichum candidans, Monilia sitophila, Scenedesmus quadricauda, Chlorella vulgaris, Nostoc muscorium, Oscillatoria limosa und Anabaena constricta.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser, Trägerstoffe wie natürliche Gesteinsmehle, z.B. Kaoline, Tonerden, Talkum, Kreide und synthetische Gesteinsmehle (z.B. hochdisperse Kieselsäure, Silikate), Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die Formulierungen enthalten im allgemeinen zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR/HPLC/GC-Spektrum) eingesetzt.

Als übliche Anwendungskonzentration wählt man - bezogen auf das Gewicht des zu schützenden Materials - 0,001 bis 5 Gew.-%, bevorzugt 0,01 bis 2 Gew.-% an Wirkstoff; beim Einsatz zur Wasserbehandlung, bei der Erdölförderung, in Bohr- und Schneidölen, Treibstoffen, in Schwimmbädern, Rückkühlwerken, Luftbefeuchtungsanlagen oder in der Papierindustrie sind Wirkstoffmengen von 5 bis 500 ppm ausreichend. Gebrauchsfertige Desinfektionsmittellösungen enthalten z.B. 0,5 bis 10 Gew.-% an Wirkstoff.

Beispiele für solche Zubereitungen sind:
I. eine Lösung aus 90 Gew.-Teilen der Verbindung Nr. 7 und 10 Gew.-Teilen N-Methyl-α-pyrrolidon, die zur Anwendung in Form kleinster Tropfen geeignet ist;
II. eine Mischung aus 20 Gew.-Teilen der Verbindung Nr. 2, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.
III. eine wäßrige Dispersion aus 20 Gew.-Teilen der Verbindung Nr. 4, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.
IV. eine wäßrige Dispersion aus 20 Gew.-Teilen der Verbindung Nr. 3, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;
V. eine in einer Hammermühle vermahlene Mischung aus 80 Gew.-Teilen der Verbindung Nr. 1, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;
VI. eine innige Mischung aus 3 Gew.-Teilen der Verbindung Nr. 5 und 97 Gew.-Teilen feinteiligem Kaolin. Dieses Stäubemittel enthält 3 Gew.-% Wirkstoff;
VII. eine innige Mischung aus 30 Gew.-Teilen der Verbindung Nr. 6, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;
VIII. eine stabile wäßrige Dispersion aus 40 Gew.-Teilen der Verbindung Nr. 4, 10 Gew.-Teilen des Natriumsalzes eines Phenosulfonsäure harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung Nr. 5, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehyd Kondensates und 68 Gew.-Teilen eines paraffinischen Mineralöls;
X. eine in einer Hammermühle vermahlene Mischung aus 10 Gew.-Teilen der Verbindung Nr. 1, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Wirkstoffe wirken für sich allein als schaumarme Biozide. Eine bedeutende Steigerung der Wirkung dieser Verbindungen enthaltender biozider Zubereitungen wird erzielt, wenn man ihnen noch Tri-C₆- bis C₁₂-alkylmethylammoniumsalze, vorzugsweise in Mengen von 20 bis 40 Gew.-%, bezogen auf das Gewicht der Verbindungen der allgemeinen Formel I, zusetzt.

Die Wirkstoffe können auch mit anderen bekannten Mikrobiziden gemischt werden. In vielen Fällen erhält man dabei einen synergistischen Effekt, d.h. die mikrobizide Wirksamkeit der Mischung ist größer als die der (addierten) Wirksamkeiten der Einzelkomponenten.

Die Zumischung der bekannten Mikrobizide zu den neuen Substanzen kann in einem Gewichtsverhältnis von 1:100 bis 100:1 erfolgen.

Solche Wirkstoffe sind beispielsweise:
2-(Thiocyanomethylthio)-benzthiazol 1-[2-(2,4-Dichlorphenyl)-2-(2-propenyl-oxy)-ethyl]-1H-imidazol 2,4,5,6-Tetrachlor-isophthalodinitril Methylenbisthiocyanat Tributylzinnoxid, -naphthenat, -benzoat, -salicylat Mercaptobenzthiazol 1,2-Benzisothiazolon und seine Alkalisalze Alkaliverbindungen des N'-Hydroxy-N-cyclohexyl-diazeniumoxids 2-(Methoxy-carbonylamino)-benzimidazol 2-Methyl-3-oxo-5-chlor-thiazolin-3-on Trihydroxymethyl-nitro-methan Glutardialdehyd Chloracetamid Polyhexamethylenbisguanide 5-Chlor-2-methyl-4-isothiazolin-3-on + Magnesiumsalze 3,5-Dimethyltetrahydro-1,3,5-2H-thiadiazin-2-thion Hexahydrotriazin N,N-Methylolchloracetamid 2-n-Octyl-4-isothiazol-in-3-on Oxazolidine Bisoxazolidine 2,5-Dihydro-2,5-dialkoxy-2,5-dialkylfurane Diethyl-dodecyl-benzyl-ammoniumchlorid Dimethyl-octadecyl-dimethylbenzyl-ammoniumchlorid Dimethyl-didecyl-ammoniumchlorid Dimethyl-didodecyl-ammoniumchlorid Trimethyl-tetradecylammoniumchlorid Benzyl-dimethyl-alkyl-(C₁₂-C₁₈)-ammoniumchlorid Dichlorbenzyl-dimethyl-dodecyl-ammoniumchlorid Cetylpyridiniumchlorid Cetylpyridiniumbromid Cetyl-trimethyl-ammoniumchlorid Laurylpyridiniumchlorid Laurylpyridiniumbisulfat Benzyl-dodecyl-di(beta-oxyethyl)-ammoniumchlorid Dodecylbenzyl-trimethyl-ammoniumchlorid n-Alkyl-dimethyl-benzyl-ammoniumchlorid (Alkylrest: 40 % C₁₂, 50 % C₁₄, 10 % C₁₆) Lauryl-dimethyl-ethyl-ammoniumethylsulfat n-Alkyl-dimethyl-(1-naphthylmethyl)-ammoniumchlorid (Alkylrest: 98 % C₁₂, 2 % C₁₄) Cetyldimethylbenzylammoniumchlorid Lauryldimethylbenzylammoniumchlorid

Weitere mögliche Mischungspartner sind beispielsweise:
1,3-Dimethylol-5,5-dimethylhydantoin Dimethylolharnstoff Tetramethylolacetylendiharnstoff Dimethylolglyoxalmonourein Hexamethylentetramin Glyoxal Glutardialdehyd N-Methylol-chloracetamid 1-(Hydroxymethyl)-5,5-dimethyl-hydantoin 1,3-Bis-(hydroxymethyl)-5,5-dimethylhydantoin Imidazolidinylharnstoff 1-(3-Chlorallyl)-3,5,7-triaza-1-azonia-adamantan-chlorid 1,3-Bis-(β-ethylhexyl)-5-methyl-5-amino-hexahydropyrimidin 1,3,5-Tris-(hydroxyethyl)-1,3,5-hexahydrotriazin 1,2-Dibrom-2,4-dicyanobutan 5-Brom-5-nitro-1,3-dioxan 2-Brom-2-nitropropandiol 1,1'-Hexamethylen-bis-[5-(4-chlorphenyl)-biguanid] 4,4-Diaminodiphenoxypropan 2-Brom-2-nitro-propan-1,3-diol Sorbinsäure und ihre Salze p-Hydroxybenzoesäure und ihre Ester und Salze Zink-2-pyridinethiol-N-oxid 2-[(Hydroxylmethyl)amino]-ethanol Dithio-2,2'-bis(benzmethyl-amid) 5-Chlor-2-(2,4-dichlorphenoxy)-phenol Thio-bis-(4-chlorphenol) o-Phenyl-phenol Chlormethyl-dijodmethylsulfon p-Chlorphenyl-3-jodpropargyl-formal

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I genutzt. Die so erhaltenen Verbindungen sind in den anschließenden Tabellen mit physikalischen Daten aufgeführt.
1. N-(2-(1-Methylethyl)phenyl)-1-methyl-3-trifluormethylpyrazol-4-carbonsäureamid 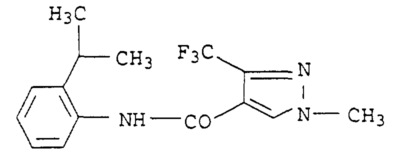
   a) 1-Methyl-3-trifluormethylpyrazol-4-carbonsäureethylester
      Zu einer Lösung von 1,38 g Methylhydrazin in 30 ml Ethanol tropft man bei -35 bis -40°C 7,20 g Ethoxymethyl-4,4,4-trifluoracetessigsäureethylester und rührt 1 Stunde bei 0°C und 1 h bei 40°C nach. Nach Verdampfen des Lösungsmittel isoliert man 6,02 g Kristalle vom Fp. 52 - 54°C, der zu 85 % aus dem obigen Ester und zu 15 % aus 1-Methyl-5-trifluormethylpyrazol-4-carbonsäureethylester besteht.
   b) 1-Methyl-3-trifluormethylpyrazol-4-carbonsäure
      Zu 7,4 g Natriumhydroxid in 187 ml Wasser gibt man 41,5 g des obigen Rohprodukts aus a) setzt 3 ml Ethanol zu und rührt 12 Stunden bei Raumtemperatur. Nach Abfiltrieren von wenig Rückstand säuert man das Filtrat mit konzentrierter Salzsäure auf pH 3 an. Nach Absaugen des Produkts, Waschen mit kaltem Wasser und Trocknen isoliert man 29,0 g der obigen Säure vom Fp. 201 - 202°C.
   c) 1-Methyl-3-trifluormethylpyrazol-4-carbonsäure-2'-sec.-butylanilid
      Zu einer Lösung von 2,91 g der Säure aus b) und 1,60 g Triethylamin in 30 ml Dichlormethan tropft man bei 0°C 1,90 g Thionylchlorid zu und rührt 3 Stunden bei 0°C nach. Anschließend tropft man bei gleicher Temperatur eine Mischung von 2,43 g 2-sec.-Butylanilin und 1,60 g Triethylamin zu und rührt 12 Stunden bei Raumtemperatur nach. Nach Waschen des Ansatzes mit 60 ml Wasser isoliert man nach Trocknen und Verdampfen des Lösungsmittels 4,30 g Rohprodukt, aus dem man nach Umkristallisation aus Cyclohexan 3,50 g des obigen Anilids vom Fp. 126 - 129°C erhält.

### Beispiel 2

a) Zu einer Lösung von 1,5 g 2-iso-Butylanilin und 1,0 g Triethylamin in 12 ml Tetrahydrofuran tropft man bei 0°C 2,5 g 2-Chlor-4-trifluormethyl-5-thiazolcarbonsäurechlorid (J. Het. Chem. 22, 1621 (1985)). Nach 12 Stunden Rühren bei Raumtemperatur verdünnt man das Reaktionsgemisch mit 250 ml Wasser und extrahiert zweimal mit je 70 ml Essigester. Nach Trocknen, Filtrieren und Verdampfen des Lösungsmittels sowie Anteigen des Rohprodukts mit Diisopropylether isoliert man 2,8 g 2-Chlor-4-trifluormethyl-5-thiazolcarbonsäure-2'-isobutylanilid vom Fp. 107 - 108°C.
b) In eine Lösung von 9,0 g des obigen Produkts und 0,7 g Phenol-4-sulfonsäure (65 %ig) in 100 ml Ethanol werden im Autoklav 30 ml Ammoniak aufgepreßt und bei 120°C 24 Stunden gerührt. Nach Entspannen wird der Austrag filtriert, eingeengt und das Rohprodukt zwischen 300 ml Essigester und 100 ml Wasser verteilt. Aus der organischen Phase isoliert man nach Trocknen und Verdampfen des Lösungsmittels 7,0 g 2-Amino-4-trifluormethyl-5-thiazol-carbonsäure-2'-isobutylanilid vom Fp. 193 - 196.

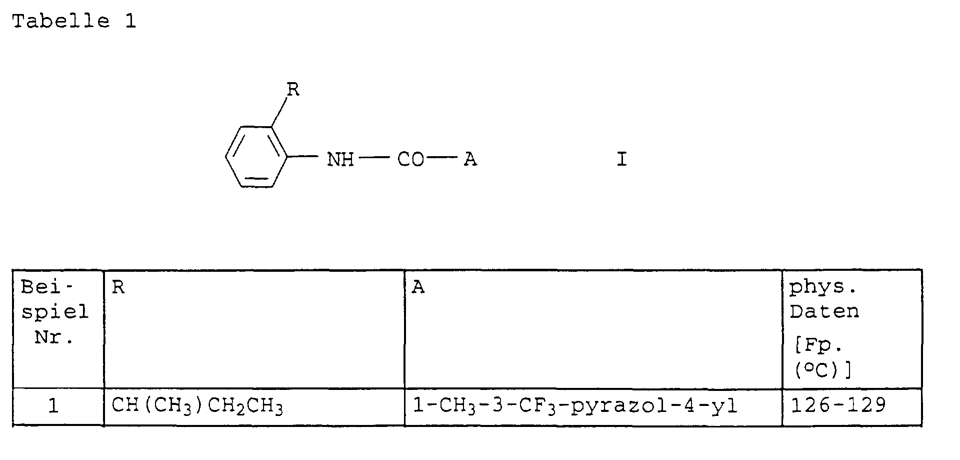

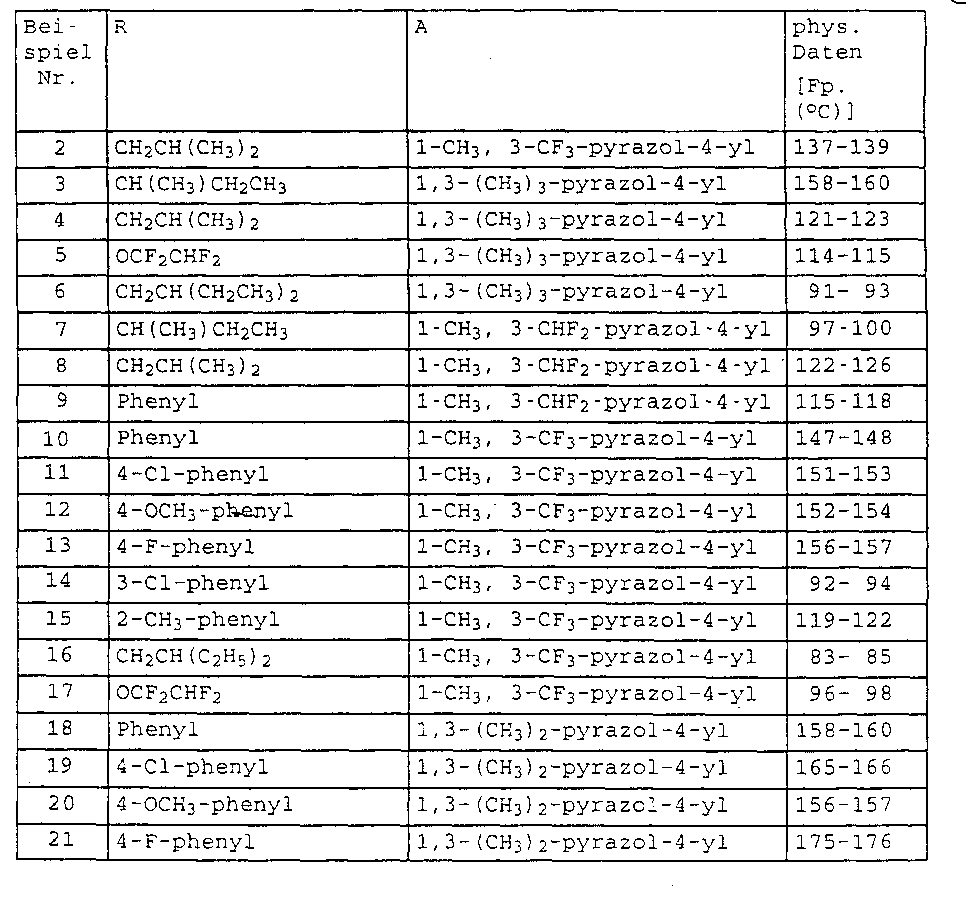

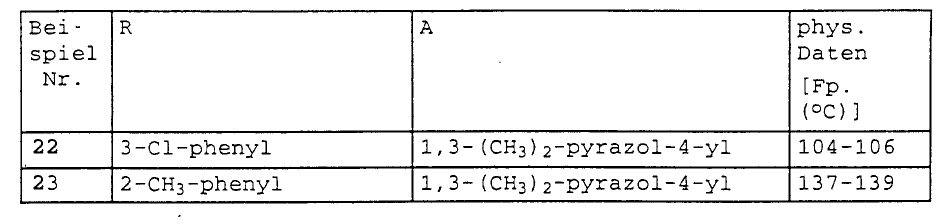

### Beispiele zur biologischen Wirkung:

### Wirksamkeit gegen Botrytis cinerea

Paprikasämlinge (Sorte: "Neusiedler Ideal Elite") mit 4-5 gut entwickelten Blättern wurden mit einer wäßrigen Suspension [80% Wirkstoff / 20% Emulgator in der Trockenmasse] des Wirkstoffs tropfnaß gespritzt. Nach dem Abtrocknen des Spritzbelags wurden die Pflanzen mit einer Konidienaufschwemmung des Pilzes Botrytis cinerea besprüht und anschließend 5 Tage bei 22-24°C und hoher Luftfeuchtigkeit aufbewahrt.

Nach dieser Zeit wiesen die nicht mit Wirkstoff vorbehandelten Kontroll-Pflanzen einen Pilzbefall von 80% auf, während die mit jeweils 500 ppm der Verbindungen Nr. 1, 3, 4, 5 und 6 behandelten Pflanzen maximal zu 15% befallen waren.

## Patentansprüche

1. Carbonsäureanilide der Formel I 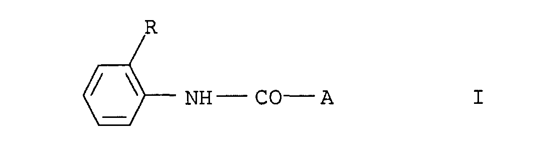 in der die Substituenten die folgende Bedeutung haben:
R C₃-C₁₂-Alkyl, C₂-C₁₂-Alkoxy, C₃-C₁₂-Alkenyl, C₃-C₁₂-Alkenyloxy, C₃-C₆-Alkinyl, C₃-C₆-Alkinyloxy, wobei diese Gruppen partiell oder vollständig halogeniert sein können;
Phenyl, welches ein bis fünf Halogenatome und/oder ein bis drei der folgenden Reste tragen kann: C₁-C₄-Alkyl, C₁-C₄-Halogenalkyl, C₁-C₄-Alkoxy, C₁-C₄-Halogenalkoxy, C₁-C₄-Alkylthio oder C₁-C₄-Halogenalkylthio;
A ein cyclischer Rest der Formel A5: 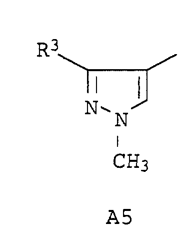 wobei
R³ C₁-C₄-Alkyl oder C₁-C₄-Halogenalkyl bedeutet.

2. Carbonsäureanilide der Formel I, gemäß Anspruch 1, in der R die in Anspruch 1 gegebene Bedeutung hat und
R³ Methyl oder C₁-Halogenalkyl bedeutet.

3. Carbonsäureanilide der Formel I, gemäß Anspruch 1, in der R die in Anspruch 1 gegebene Bedeutung hat und
R³ Methyl, Difluormethyl oder Trifluormethyl bedeutet.

4. Verfahren zur Herstellung der Verbindungen I gemäß einem der Ansprüche 1 - 3, **dadurch gekennzeichnet, daß** man ein Carbonsäurehalogenid der Formel II
Hal-CO-A II
in der Hal für ein Halogenatom steht, in an sich bekannter Weise in Gegenwart einer Base mit einem Anilin der Formel III 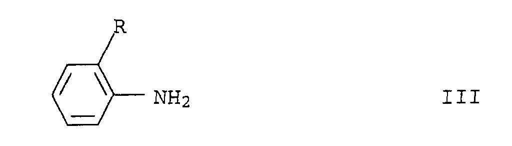 umsetzt.

5. Mittel zur Bekämpfung von Schadpilzen, enthaltend eine fungizide Menge einer Verbindung der Formel I gemäß Anspruch 1, 2 oder 3 und inerte Zusatzstoffe.

6. Verfahren zur Bekämpfung von Schadpilzen, **dadurch gekennzeichnet, daß** man die Schadpilze, ihren Lebensraum und/oder die von Schadpilzen freizuhaltenden Pflanzen oder Materialien mit einer fungizid wirksamen Menge einer Verbindung der Formel I gemäß Anspruch 1, 2 oder 3 behandelt.

7. Verwendung der Verbindungen I gemäß Anspruch 1, 2 oder 3 zur Bekämpfung von Schadpilzen.

8. Verwendung der Verbindungen I gemäß Anspruch 1, 2 oder 3 zur Bekämpfung von Botrytis.

## Claims

1. A carboxanilide of the formula I 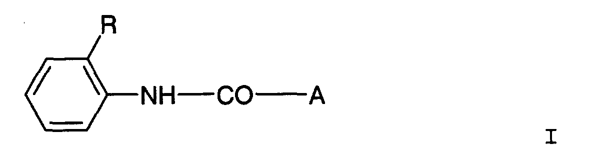 in which the substituents have the following meaning:
R is C₃-C₁₂-alkyl, C₂-C₁₂-alkoxy, C₃-C₁₂-alkenyl, C₃-C₁₂-alkenyloxy, C₃-C₆-alkynyl, C₃-C₆-alkynyloxy, where these groups can be partially or completely halogenated;
phenyl which can carry one to five halogen atoms and/or one to three of the following radicals: C₁-C₄-alkyl, C₁-C₄-haloalkyl, C₁-C₄-alkoxy, C₁-C₄-haloalkoxy, C₁-C₄-alkylthio or C₁-C₄-haloalkylthio;
A is a cyclic radical of the formula A5: 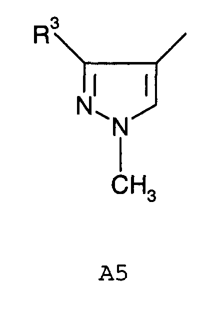 where
R³ is C₁-C₄-alkyl or C₁-C₄-haloalkyl.

2. A carboxanilide of the formula I, as claimed in claim 1, in which R has the meaning given in claim 1 and
R³ is methyl or C₁-haloalkyl.

3. A carboxanilide of the formula I, as claimed in claim 1, in which R has the meaning given in claim 1 and
R³ is methyl, difluoromethyl or trifluoromethyl.

4. A process for the preparation of the compounds I as claimed in one of claims 1 - 3, which comprises reacting a carboxylic acid halide of the formula II
Hal-CO-A II
in which Hal is a halogen atom, in a manner known per se, in the presence of a base with an aniline of the formula III

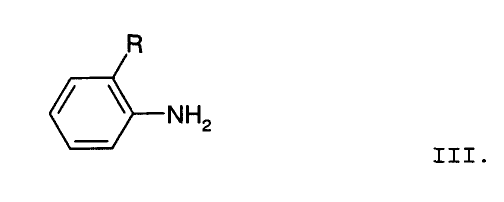

5. A composition for controlling harmful fungi, comprising a fungicidal amount of a compound of the formula I as claimed in claim 1, 2 or 3 and inert additives.

6. A procedure for controlling harmful fungi, which comprises treating the harmful fungi, their habitat and/or the plants or materials to be kept free from harmful fungi with a fungicidally active amount of a compound of the formula I as claimed in claim 1, 2 or 3.

7. The use of the compounds I as claimed in claim 1, 2 or 3 for controlling harmful fungi.

8. The use of the compounds I as claimed in claim 1, 2 or 3 for controlling botrytis.

## Revendications

1. Carboxanilides de formule I 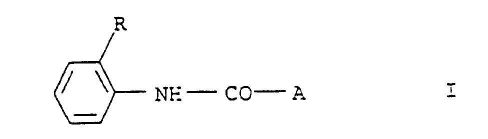 dans laquelle les symboles ont les significations suivantes :
R : un groupe alkyle en C₃-C₁₂, alcoxy en C₂-C₁₂, alcényle en C₃-C₁₂, alcényloxy en C₃-C₁₂, alcynyle en C₃-C₆, alcynyloxy en C₃-C₆, tous ces groupes pouvant être halogénés en totalité ou en partie ;
un groupe phényle qui peut porter un à cinq atomes d'halogènes et/ou un à trois des substituants suivants : alkyle en C₁-C₄, halogénoalkyle en C₁-C₄, alcoxy en C₁-C₄, halogénoalcoxy en C₁-C₄, alkylthio en C₁-C₄, ou halogénoalkylthio en C₁-C₄,
A : un radical cyclique de formule A5 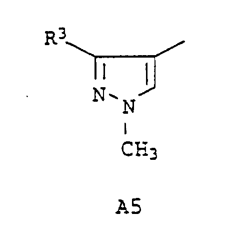 dans laquelle
R³ représente un groupe alkyle en C₁-C₄ ou halogénoalkyle en C₁-C₄.

2. Carboxanilides de formule I selon la revendication 1, dans laquelle R a les significations indiquées dans la revendication 1 et
R³ représente un groupe méthyle ou halogénoalkyle en C₁.

3. Carboxanilides de formule I selon la revendication 1, dans laquelle R a les significations indiquées dans la revendication 1 et
R³ représente un groupe méthyle, difluorométhyle ou trifluorométhyle.

4. Procédé pour la préparation des composés I selon l'une des revendications 1 à 3, **caractérisé par le fait que** l'on fait réagir un halogénure d'acide carboxylique de formule II
Hal-CO-A II
dans laquelle Hal représente un atome d'halogène, de manière connue en soi et en présence d'une base, avec une aniline de formule III

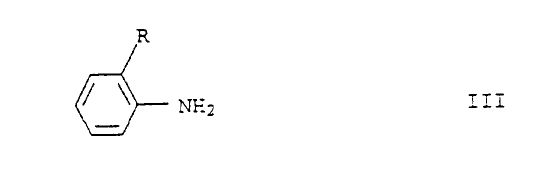

5. Produit pour combattre les mycètes nuisibles, contenant une quantité fongicide d'un composé de formule I selon la revendication 1, 2 ou 3 et des additifs inertes.

6. Procédé pour combattre les mycètes nuisibles, **caractérisé par le fait que** l'on traite les mycètes nuisibles, leur habitat et/ou les végétaux ou matériaux à protéger contre les mycètes nuisibles par une quantité fongicide efficace d'un composé de formule I selon la revendications 1, 2 ou 3.

7. Utilisation des composés I selon la revendications 1, 2 ou 3 pour la lutte contre les mycètes nuisibles.

8. Utilisation des composés I selon la revendications 1, 2 ou 3 pour la lutte contre Botrytis.
